# EUROPÄISCHE PATENTSCHRIFT

(11) **EP 3 593 835 B1**
(45) Veröffentlichungstag und Bekanntmachung des Hinweises auf die Patenterteilung: **10.12.2025**
(21) Anmeldenummer: 19177873.7
(22) Anmeldetag: 03.06.2019
(51) Int. Cl.: A61M 5/145, A61M 5/168, A61M 5/152, G01L 7/08, G01L 13/02

(54) **INFUSIONSANORDNUNG ZUR VERABREICHUNG EINES MEDIZINISCHEN FLUIDS**
INFUSION ASSEMBLY FOR ADMINISTERING A MEDICAL FLUID
DISPOSITIF DE PERFUSION DESTINÉ À L'ADMINISTRATION D'UN FLUIDE MÉDICAL

(30) Priorität: 10.07.2018 DE 102018211392
(43) Veröffentlichungstag der Anmeldung: 15.01.2020
(73) Patentinhaber: B. Braun Melsungen AG, 34212 Melsungen (DE)
(72) Erfinder: Haslbeck, Karsten, 34212 Melsungen (DE); Wildhagen, Jens, 30659 Hannover (DE)
(74) Vertreter: Patentanwälte Ruff, Wilhelm, Beier, Dauster & Partner mbB

(56) Entgegenhaltungen:
- US-A- 3 603 148
- US-A- 5 655 568
- US-A- 6 122 972
- US-A1- 2005 267 413
- US-A1- 2009 113 996

## Beschreibung

Die Erfindung betrifft eine Infusionsanordnung zur Verabreichung eines medizinischen Fluids, aufweisend eine medizinische Pumpvorrichtung mit einer elastomeren Membran, die ein Pumpvolumen zur Aufnahme und Förderung des medizinischen Fluids ausbildet, wobei die elastomere Membran in einem wenigstens teilweise mit dem medizinischen Fluid befüllten Füllzustand des Pumpvolumens elastisch gedehnt ist und somit einen Förderdruck auf das Pumpvolumen bewirkt, und aufweisend eine einends mit dem Pumpvolumen wirkverbundene Infusionsleitung, die andernends zur fluidleitenden Verbindung mit einem Patientenzugang vorgesehen ist und einen Hauptfluidkanal zur Überleitung des medizinischen Fluids von dem Pumpvolumen zu dem Patientenzugang ausbildet, und aufweisend eine mit der Infusionsleitung wirkverbundene mechanische Kontrolleinrichtung, die zur Funktionskontrolle der Pumpvorrichtung eingerichtet ist.

Eine derartige Infusionsanordnung ist aus der WO 2015/110387 A1 bekannt und zur Verabreichung eines medizinischen Fluids im Rahmen einer Infusionstherapie vorgesehen. Die bekannte Infusionsanordnung weist eine medizinische Pumpvorrichtung auf, die in Form einer medizinischen Elastomerpumpe mit einer ballonartig elastisch dehnbaren Membran zur Aufnahme und Förderung des medizinischen Fluids ausgebildet ist. Zudem weist die bekannte Infusionsanordnung eine mit einem Pumpvolumen der Membran wirkverbundene Infusionsleitung auf. Die Infusionsleitung bildet einen Hauptfluidkanal zur Überleitung des medizinischen Fluids von dem Pumpvolumen zu einem patientenseitigen Zugang aus. Zur Funktionskontrolle der Pumpvorrichtung weist die bekannte Infusionsanordnung eine mechanische Kontrolleinrichtung auf. Die Kontrolleinrichtung weist ein mittels eines Kanalabzweigs fluidleitend mit dem Hauptfluidkanal verbundenes Messreservoir auf. Zur Funktionskontrolle wird der Hauptfluidkanal hinter dem Kanalabzweig mittels einer Klemme manuell abgeklemmt. Bei ordnungsgemäßer Funktionsweise der Pumpvorrichtung bewirkt dies einen Druckanstieg in dem Hauptfluidkanal, was zu einem druckgetriebenen Einströmen von medizinischem Fluid in das Messreservoir führt. Funktioniert die Pumpe nicht oder ist der Hauptfluidkanal vor dem Kanalabzweig blockiert, gelangt hingegen kein medizinischen Fluid in das Messreservoir. In Abhängigkeit davon, ob medizinisches Fluid in das Messreservoir einströmt oder nicht, kann ein Anwender somit auf die Funktionstüchtigkeit der Pumpvorrichtung schließen. Bei der bekannten Infusionsanordnung erfordert die Funktionskontrolle somit stets eine zeitweise Unterbrechung der Fluidverabreichung. Zudem liefert die Funktionskontrolle lediglich eine rein qualitative Information, nämlich ob eine Pumpförderung stattfindet oder nicht.

Aufgabe der Erfindung ist es, eine Infusionsanordnung der eingangs genannten Art zu schaffen, die eine verbesserte Funktionskontrolle der Pumpvorrichtung ermöglicht, wobei insbesondere eine Unterbrechung der Fluidverabreichung vermieden und eine quantitative Information hinsichtlich der Förderfunktion der Pumpvorrichtung ermöglicht sein soll.

Diese Aufgabe wird dadurch gelöst, dass die Kontrolleinrichtung ein mit dem Hauptfluidkanal wirkverbundenes Differenzdruckmesselement aufweist und derart gestaltet ist, dass ein entlang eines Kanalabschnitts des Hauptfluidkanals ausgebildeter Differenzdruck mittels des Differenzdruckmesselements erfassbar ist und in Abhängigkeit des Differenzdrucks eine Förderrate des medizinischen Fluids entlang des Hauptfluidkanals anzeigbar ist. Die erfindungsgemäße Lösung ermöglicht eine differenzdruckabhängige Funktionskontrolle in einem laufenden Förderbetrieb der Pumpvorrichtung und damit eine unterbrechungsfreie, kontinuierliche Fluidverabreichung. Dadurch kann auf eine zeitweise Unterbrechung des Hauptfluidkanals und somit auf eine zeitweise Unterbrechung der Infusionstherapie verzichtet werden. Das Differenzdruckmesselement dient einer Erfassung des entlang des Kanalabschnitts ausgebildeten Differenzdrucks. Ein solcher Differenzdruck kann aufgrund zwangsläufig vorhandener reibungsbedingter Strömungsverluste und/oder aufgrund eines in dem Kanalabschnitt angeordneten Fluiddrosselelements ausgebildet sein. Liegt eine Funktionsstörung der Pumpvorrichtung vor oder ist der Kanalabschnitt blockiert, strömt hingegen kein medizinisches Fluid durch den Kanalabschnitt und es bildet sich folglich kein solcher Differenzdruck aus. Dabei ist die Kontrolleinrichtung derart eingerichtet, dass in Abhängigkeit des mittels des Differenzdruckmesselements ermittelten Differenzdrucks die Förderrate des medizinischen Fluids entlang des Hauptfluidkanals anzeigbar ist. Die Förderrate kann auch als Volumenstrom, Massenstrom oder Durchflussrate bezeichnet werden. Dadurch ermöglicht die Kontrolleinrichtung eine qualitative Information hinsichtlich der Förderfunktion der Pumpvorrichtung. Infolgedessen kann ein Anwender der Infusionsanordnung zusätzlich zu einer grundlegenden Kontrolle der Pumpfunktion auch die Förderrate des medizinischen Fluids kontrollieren. Dadurch wird eine Unter- und/oder Überdosierung des medizinischen Fluids vermieden, was letztlich eine verbesserte Infusionstherapie und eine erhöhte Patientensicherheit ermöglicht. Die mechanische Kontrolleinrichtung ist vorzugsweise derart gestaltet, dass auf eine externe Energieversorgung verzichtet werden kann. Die medizinische Pumpvorrichtung ist vorzugsweise in Form einer im Bereich der Medizintechnik allgemein bekannten medizinischen Elastomerpumpe ausgebildet, die auch als elastomerische Infusionspumpe bezeichnet werden kann. Die elastomere Membran bildet das Pumpvolumen und damit eine Art Hohlraum aus. Die elastomere Membran kann somit auch als elastomere Hohlmembran bezeichnet werden.

Erfindungsgemäß weist die Kontrolleinrichtung einen Bypass-Kanal auf, der einends mittels eines ersten Kanalabzweigs und andernends mittels eines zweiten Kanalabzweigs jeweils fluiddruckübertragend mit dem Hauptfluidkanal wirkverbunden ist, wobei das Differenzdruckmesselement in dem Bypass-Kanal angeordnet ist. Der Bypass-Kanal ist vorzugsweise in Form einer Schlauchleitung ausgebildet. Die Kanalabzweige können einstückig an der Infusionsleitung angeordnet oder nachträglich an diese angefügt sein. Der Kanalabschnitt erstreckt sich vorzugsweise zwischen dem ersten Kanalabzweig und dem zweiten Kanalabzweig. Vereinfacht ausgedrückt dient der Bypass-Kanal einem Abgreifen des Differenzdrucks zwischen dem ersten und dem zweiten Kanalabzweig. Vorzugsweise dient der Bypass-Kanal ausschließlich der Erfassung des Differenzdrucks. Eine Förderung des medizinischen Fluids entlang des Bypass-Kanals ist vorzugsweise nicht vorgesehen.

Erfindungsgemäß weist das Differenzdruckmesselement wenigstens ein mechanisches Messglied auf, das mittels einer Differenzdruckbeaufschlagung federelastisch auslenkbar ist. Das mechanische Messglied ist fluiddruckübertragend mit dem Hauptfluidkanal wirkverbunden. Sofern ein Bypass-Kanal vorgesehen ist, ist das mechanische Messglied vorzugsweise mit dem Bypass-Kanal fluiddruckübertragend wirkverbunden. Das mechanische Messglied kann beispielsweise in Form eines hydraulisch auslenkbaren federbelasteten Kolbens, einer Membran oder dergleichen ausgebildet sein.

Erfindungsgemäß weist das mechanische Messglied wenigstens eine federelastische Membran auf. Dies ist eine besonders robuste und zuverlässige Ausgestaltung der Erfindung.

Erfindungsgemäß weist die Kontrolleinrichtung ein mit dem Differenzdruckmesselement wirkverbundenes Zeigerelement auf, das zur Anzeige der Förderrate in Abhängigkeit des Differenzdrucks eingerichtet ist. Das Zeigerelement kann mittels einer mechanischen Übersetzung mit dem Differenzdruckmesselement wirkverbunden sein. Auf diese Weise kann eine differenzdruckbedingte Auslenkung des Differenzdruckmesselements, insbesondere die federelastische Auslenkung des mechanischen Messglieds des Differenzdruckmesselements, in eine Zeigerbewegung des Zeigerelements übersetzt werden. Dem Zeigerelement ist vorzugsweise eine Skale zum Ablesen der Förderrate zugeordnet.

Erfindungsgemäß weist die Kontrolleinrichtung wenigstens eine fluidgefüllte Fluidkammer auf, wobei das Zeigerelement in der Fluidkammer schwimmbeweglich angeordnet ist. Die Fluidkammer ist vorzugsweise hydraulisch mit dem Hauptfluidkanal wirkverbunden. Sofern ein Bypass-Kanal vorgesehen ist, ist die Fluidkammer vorzugsweise hydraulisch mit dem Bypass-Kanal verbunden. Die Fluidkammer ist vorzugsweise wenigstens abschnittsweise durchsichtig ausgebildet, so dass ein Anwender eine differenzdruckbedingte Schwimmbewegung des Zeigerelements innerhalb der Fluidkammer einsehen kann.

In weiterer Ausgestaltung der Erfindung ist das Zeigerelement in Form eines Schwimmers oder eines eingefärbten Öltropfens ausgebildet. Dabei dient die Einfärbung des Öltropfens einer verbesserten visuellen Wahrnehmbarkeit der jeweiligen Schwimmposition des Öltropfens innerhalb der Fluidkammer. Hierdurch kann eine verbesserte Ablesbarkeit der Kontrolleinrichtung erreicht werden.

In weiterer Ausgestaltung der Erfindung weist die Kontrolleinrichtung ein zwischen dem ersten Kanalabzweig und dem zweiten Kanalabzweig in dem Hauptfluidkanal angeordnetes erstes Fluiddrosselelement auf. Das Fluiddrosselelement ist somit vorzugsweise innerhalb des Kanalabschnitts angeordnet. Das Fluiddrosselelement dient einer Beeinflussung des Differenzdrucks. Im Vergleich zu einer ungedrosselten Ausgestaltung, bei der der Differenzdruck infolge zwangsläufig vorhandener Strömungsverluste innerhalb des Kanalabschnitts ausgebildet ist, bewirkt das Fluiddrosselelement einen vergleichsweise höheren Differenzdruck. Dies erlaubt insbesondere eine weniger messempfindliche Ausgestaltung des Differenzdruckmesselements. Das Fluiddrosselelement kann in Form einer Verengung des Kanalabschnitts oder als separates fluidtechnisches Bauelement ausgebildet und in den Kanalabschnitt eingebracht sein.

In weiterer Ausgestaltung der Erfindung ist stromabwärts des zweiten Kanalabzweigs ein zweites Fluiddrosselelement in dem Hauptfluidkanal angeordnet. Das zweite Fluiddrosselelement dient insbesondere einer Druckminderung des zu verabreichenden medizinischen Fluids, so dass dieses mit einem vorherbestimmten Ausgangsdruck ausgehend von der Infusionsleitung in den Patientenzugang abgegeben werden kann. Dabei meint stromabwärts, dass das zweite Fluiddrosselelement - in Bezug auf eine bestimmungsgemäße Förderrichtung der Pumpvorrichtung - hinter dem zweiten Kanalabzweig angeordnet ist. Das zweite Fluiddrosselelement kann in Form einer Verengung des Hauptfluidkanals ausgebildet oder als separates fluidtechnisches Bauelement in dem Hauptfluidkanal angeordnet sein.

In weiterer Ausgestaltung der Erfindung weist das erste Fluiddrosselelement im Vergleich zu dem zweiten Fluiddrosselelement eine, vorzugsweise 1,5-fach bis 15-fach, geringere Drosselwirkung auf. Dementsprechend weist das erste Fluiddrosselelement eine im Vergleich zu dem zweiten Fluiddrosselelement verringerte, vorzugsweise signifikant verringerte, Drosselwirkung auf. Es hat sich gezeigt, dass diese Ausgestaltung der Erfindung eine besonders vorteilhafte Ermittelbarkeit der Förderrate ermöglicht. Insbesondere kann eine Linearisierung der Förderratenermittlung gegenüber Druckschwankungen der medizinischen Pumpvorrichtung ermöglicht werden.

Weitere Vorteile und Merkmale der Erfindung ergeben sich aus den Ansprüchen sowie aus der nachfolgenden Beschreibung eines bevorzugten Ausführungsbeispiels der Erfindung, das anhand der Zeichnungen dargestellt ist.
- Fig. 1: zeigt in schematischer, teilweise aufgeschnittener Darstellung eine Ausführungsform einer erfindungsgemäßen Infusionsanordnung und
- Fig. 2: die Infusionsanordnung nach Fig. 1 in einer stark vereinfachten schematischen Darstellung.

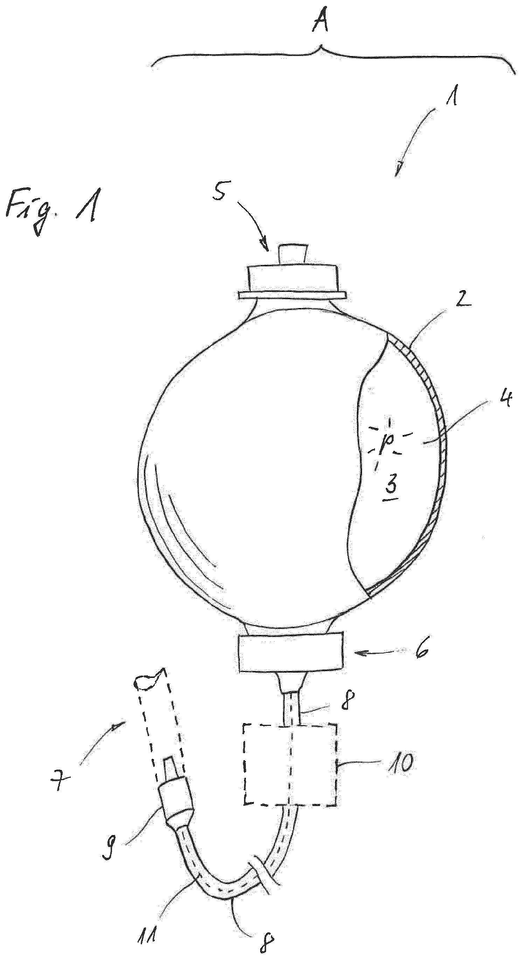

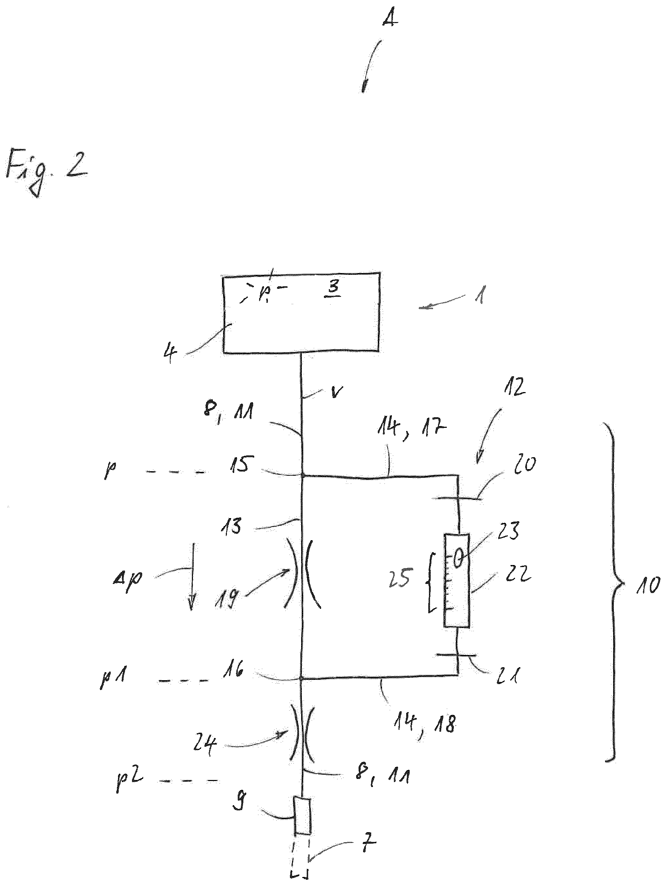

Eine Infusionsanordnung A nach den Fig. 1 und 2 ist zur Verabreichung eines medizinischen Fluids 4 im Rahmen einer ambulanten und/oder stationären Infusionstherapie vorgesehen. Die Infusionsanordnung A weist eine medizinische Pumpvorrichtung 1 auf, die auch als elastomerische Infusionspumpe oder medizinische Elastomerpumpe bezeichnet werden kann. Die medizinische Pumpvorrichtung 1 weist eine elastomere Membran 2 auf, die ein Pumpvolumen 3 zur Aufnahme und Förderung des medizinischen Fluids 4 ausbildet. Anhand Fig. 1 ist die medizinische Pumpvorrichtung 1 in einem wenigstens teilweise mit dem medizinischen Fluid 4 befüllten Füllzustand gezeigt. In diesem Zustand ist die elastomere Membran 2 infolge einer Einwirkung des medizinischen Fluids 4 ballonartig weichelastisch gedehnt. Die Membran 2 ist anhand Fig. 1 aus zeichnerischen Gründen mit einer überhöhten Wandungsstärke dargestellt. In einem nicht mit medizinischem Fluid 4 befüllten Zustand ist die Membran 2 demgegenüber schlaff oder jedenfalls weniger stark elastisch gedehnt. Zum Befüllen des Pumpvolumens 3 bzw. der Membran 2 mit medizinischem Fluid 4 ist ein wiederverschließbarer Einfüllstutzen 5 vorgesehen, der auf grundsätzlich bekannte Weise fluiddicht an die Membran 2 angeschlossen ist.

Die elastisch gedehnte Membran 2 bewirkt einen Förderdruck p auf das Pumpvolumen 3. Mittels des auf diese Weise bewirkten Förderdrucks p ist das medizinische Fluid 4 über einen fluiddicht an die Membran 2 angeschlossenen Auslassstutzen 6 aus dem Pumpvolumen 3 in eine einends fluidleitend mit dem Pumpvolumen 3 wirkverbundene Infusionsleitung 8 förderbar. Die Infusionsleitung 8 ist zu diesem Zweck einends an ihrem dem Pumpvolumen 3 zugewandten Stirnende unlösbar mit dem Auslassstutzen 6 verbunden. An ihrem gegenüberliegenden Stirnende weist die Infusionsleitung 8 einen Anschluss 9 auf, der vorliegend in Form eines Luer-Anschlusses ausgebildet ist. Bei einer nicht dargestellten Ausführungsform ist der Anschluss in Form eines NRFit-Anschlusses ausgebildet. Der Luer-Anschluss 9 ist zur Verbindung mit einem Patientenzugang 7 vorgesehen. Der Patientenzugang 7 ist anhand Fig. 1 lediglich stark vereinfacht schematisch und teilweise abgeschnitten dargestellt. Auf diese Weise bildet die Infusionsleitung 8 einen Hauptfluidkanal 11 zur Überleitung des medizinischen Fluids 4 von dem Pumpvolumen 3 zu dem Patientenzugang 7 aus. Der Hauptfluidkanal 11 ist anhand Fig. 1 schematisch strichliert dargestellt.

Die Pumpvorrichtung 1 ist vorliegend derart dimensioniert, dass sie ohne weiteres von einem Patienten am Körper getragen und ohne eine externe Energieversorgung im Rahmen einer ambulanten Therapie verwendbar ist. Die Pumpvorrichtung 1 ist dementsprechend leicht und kompakt dimensioniert, wobei das Pumpvolumen 3 vorliegend nominal mit 400 ml bemessen ist. Es versteht sich, dass das Pumpvolumen 3 auch hiervon abweichend, beispielsweise mit 50 ml bis 750 ml, bemessen sein kann.

Wie weiter anhand Fig. 1 ersichtlich ist, weist die Infusionsanordnung A eine mit der Infusionsleitung 8 wirkverbundene mechanische Kontrolleinrichtung 10 auf. Die Kontrolleinrichtung 10 ist zur Funktionskontrolle der medizinischen Pumpvorrichtung 1 eingerichtet und zu diesem Zweck mit der Infusionsleitung 8 wirkverbunden. Anhand Fig. 1 ist die Kontrolleinrichtung 10 lediglich schematisch angedeutet. Weitere konstruktive und funktionelle Merkmale der Kontrolleinrichtung 10 sind anhand Fig. 2 ersichtlich.

Die Kontrolleinrichtung 10 weist ein mit dem Hauptfluidkanal 11 wirkverbundenes Differenzdruckmesselement 12 auf und ist derart gestaltet, dass ein entlang eines Kanalabschnitts 13 des Hauptfluidkanals 11 ausgebildeter Differenzdruck Δp mittels des Differenzdruckmesselements 12 erfassbar und in Abhängigkeit des Differenzdrucks Δp eine Förderrate v des medizinischen Fluids 4 entlang des Hauptfluidkanals 11 anzeigbar ist.

Vorliegend weist die mechanische Kontrolleinrichtung 10 zu diesem Zweck einen Bypass-Kanal 14 auf. Der Bypass-Kanal 14 ist einends mittels eines ersten Kanalabzweigs 15 und andernends mittels eines zweiten Kanalabzweigs 16 jeweils fluiddruckübertragend mit dem Hauptfluidkanal 11 wirkverbunden. Dabei ist das Differenzdruckmesselement 12 vorliegend in dem Bypass-Kanal 14 angeordnet. Der Bypass-Kanal ist vorliegend in Form einer Schlauchleitung 14 ausgebildet, die einen ersten Leitungsabschnitt 17 und einen zweiten Leitungsabschnitt 18 aufweist. Der erste Leitungsabschnitt 17 stellt eine fluiddruckübertragenden Wirkverbindung zwischen dem ersten Kanalabzweig 15 und dem Differenzdruckmesselement 12 her. Der zweite Leitungsabschnitt stellt eine fluiddruckübertragende Wirkverbindung zwischen dem zweiten Kanalabzweig 16 und dem Differenzdruckmesselement 12 her. Der Kanalabschnitt 13 ist fluidstromseitig zwischen dem ersten Kanalabzweig 15 und dem zweiten Kanalabzweig 16 erstreckt.

Bei einer Förderung des medizinischen Fluids 4 mittels der medizinischen Pumpvorrichtung 1 bildet sich entlang des Hauptfluidkanals 11 ein Druckgefälle aus. Dieses Druckgefälle kann jedenfalls anteilig durch einen zwangsläufig vorhandenen Strömungswiderstand der Infusionsleitung 8 ausgebildet sein. Zudem weist die Kontrolleinrichtung 10 vorliegend ein zwischen dem ersten Kanalabzweig 15 und dem zweiten Kanalabzweig 16 in dem Hauptfluidkanal 11 angeordnetes Fluiddrosselelement 19 auf. Das Fluiddrosselelement 19 trägt zu dem vorbeschriebenen Druckgefälle bei und/oder beeinflusst dieses in Wesentlichem Umfang. Das Fluiddrosselelement 19 ist vorliegend in Form eines fluidtechnischen Steuerelements ausgebildet. Bei einer nicht dargestellten Ausführungsform kann das Fluiddrosselelement in Form einer Verengung des Strömungsquerschnitts einstückig an der Infusionsleitung 8 ausgebildet sein.

Vereinfacht ausgedrückt wird mittels des Bypass-Kanals 14 der Fluiddruck vor und hinter dem Fluiddrosselelement 19 abgegriffen. Stromaufwärts des Fluiddrosselelements 19 liegt - jedenfalls näherungsweise - der Förderdruck p an. Stromabwärts des Fluiddrosselelements im Bereich des zweiten Kanalabzweigs 16 liegt demgegenüber ein Fluiddruck p1 an. Der Differenzdruck Δp ermittelt sich insoweit aus der Differenz der beiden vorgenannten Drücke p und p1.

Vorliegend weist das Differenzdruckmesselement 12 wenigstens ein mechanisches Messglied 20, 21 auf, das mittels einer Differenzdruckbeaufschlagung federelastisch auslenkbar ist. Dabei sind vorliegend ein erstes Messglied 20 und ein zweites Messglied 21 vorgesehen. Das erste Messglied 20 ist stirnendseitig des ersten Leitungsabschnitts 17 angeordnet. Das zweite Messglied 21 ist stirnendseitig des zweiten Leitungsabschnitts 18 angeordnet. Die Messglieder 20, 21 sind jeweils fluiddruckübertragend mit dem Bypass-Kanal 14 wirkverbunden und jeweils in Form einer federelastisch auslenkbaren Membran 20, 21 ausgebildet. Zudem weist die Kontrolleinrichtung 10 eine fluidgefüllte Fluidkammer 22 auf. Die beiden Membranen 20, 21 sind jeweils hydraulisch mit der Fluidkammer 22 gekoppelt. In der Fluidkammer 22 ist ein Zeigerelement 23 schwimmbeweglich angeordnet. Das Zeigerelement ist vorliegend in Form eines Schwimmers 23 ausgebildet und zur Anzeige der Förderrate v in Abhängigkeit des Differenzdrucks Δp eingerichtet. Bei einer nicht dargestellten Ausführungsform kann das Zeigerelement in Form eines eingefärbten Öltropfens ausgebildet sein. Die Fluidkammer 22 ist derart hydraulisch mit den Membranen 20, 21 wirkverbunden, dass der mittels der Membranen 20, 21 erfasste Differenzdruck Δp auf das in der Fluidkammer 22 befindliche nicht näher bezeichnete Fluid übertragbar ist. Eine fluidleitende Verbindung ausgehend von dem ersten Kanalabzweig 15 über den Bypass-Kanal 14 weiter zu dem zweiten Kanalabzweig 16 ist mittels des Differenzdruckmesselements 12 und insbesondere mittels der Fluidkammer 22 vorliegend nicht bewirkt. Die Fluidkammer 22 ist jedenfalls abschnittsweise transparent ausgebildet. Zu diesem Zweck kann die Fluidkammer 22 beispielsweise aus einem durchsichtigen Kunststoff gefertigt sein. Dadurch ist eine differenzdruckbedingte Schwimmbewegung des Schwimmers 23 innerhalb der Fluidkammer 22 von einem Anwender der Infusionsanordnung A zur Funktionskontrolle der Pumpvorrichtung 1 visuell gut erkennbar. Der Fluidkammer 22 und/oder dem Zeigerelement 23 ist eine Skale 25 zugeordnet. Dabei ist die Kontrolleinrichtung 10 derart eingerichtet, dass der Schwimmer 23 in Abhängigkeit des mittels der Membranen 20, 21 auf die Fluidkammer 22 übertragenen Differenzdrucks Δp entlang der Skale 25 schwimmbeweglich ist, wobei eine Position des Schwimmers 23 in Bezug auf die Skale 25 in einem festen Verhältnis zu der Förderrate v steht.

Vorliegend ist stromabwärts des zweiten Kanalabzweigs 16 ein zweites Fluiddrosselelement 24 in dem Hauptfluidkanal 11 angeordnet. Dabei ist die Drosselwirkung des ersten Fluiddrosselelements 19 signifikant geringer als die des zweiten Fluiddrosselelements 24. Bevorzugt ist die Drosselwirkung des ersten Fluiddrosselelements 19 1,5-fach bis 15-fach geringer als die Drosselwirkung des zweiten Fluiddrosselelements 24. Stromabwärts des zweiten Fluiddrosselelements 24 liegt ein Fluiddruck p2 in dem Hauptfluidkanal 11 an. Dieser Druck entspricht dem Druck, mit dem das medizinische Fluid 4 ausgehend von der Infusionsleitung 8 in den Patientenzugang 7 abgegeben wird.

Zur Verabreichung des medizinischen Fluids 4 gelangt dieses ausgehend von dem mittels der elastisch gedehnten Membran 2 druckbeaufschlagten Pumpvolumen 3 mit dem Förderdruck p in die Infusionsleitung 8 und strömt entlang des Hauptfluidkanals 11 in den Patientenzugang 7. Dabei passiert das medizinische Fluid 4 ausgehend von dem Pumpvolumen 3 zunächst den ersten Kanalabzweig 15, tritt in den Kanalabschnitt 13 ein, durchströmt das erste Fluiddrosselelement 19, passiert den zweiten Kanalabzweig 16 und durchströmt abschließend das zweite Fluiddrosselelement 24 und den Luer-Lock-Anschluss 9.

Bei einer ordnungsgemäßen Funktion der medizinischen Pumpvorrichtung 1 erfolgt die Förderung des medizinischen Fluids 4 hierbei mit der Förderrate v, wobei sich längs des Hauptfluidkanals 11 ein Druckgefälle einstellt und im Speziellen zwischen den Kanalabzweigen 15, 16 der Differenzdruck Δp ausgebildet ist. Der Differenzdruck Δp wird über den Bypass-Kanal 14 und mittels der Membranen 20, 21 hydraulisch auf die Fluidkammer 22 übertragen.

Dabei bewegt sich der Schwimmer 23 in Abhängigkeit des Differenzdrucks Δp entlang der Skale 25. Aufgrund des physikalischen Zusammenhangs zwischen dem Differenzdruck Δp und der Förderrate v erlaubt die Kontrolleinrichtung 10 nicht lediglich eine Aussage darüber, ob ein Durchfluss vorliegt oder nicht, sondern quantifiziert diesen in Form der an der Skale 25 ablesbaren Förderrate v.

Funktioniert die Pumpvorrichtung 1 demgegenüber nicht ordnungsgemäß und bewirkt keinen Förderdruck p, stellt sich folglich auch keine Förderung des medizinischen Fluids 4 entlang der Infusionsleitung 8 ein. Stattdessen ruht das medizinische Fluid 4 innerhalb des Hauptfluidkanals 11. Dementsprechend beträgt der Differenzdruck Δp null. Dies schlägt sich in der Position des Schwimmers 23 relativ zu der Skale 25 nieder. Eine Fehlfunktion der Pumpvorrichtung 1 und/oder eine nicht anforderungsgerechte Förderrate ist somit von einem Anwender der Infusionsanordnung A leicht erkennbar.

## Patentansprüche

1. Infusionsanordnung (A) zur Verabreichung eines medizinischen Fluids (4), aufweisend eine medizinische Pumpvorrichtung (1) mit einer elastomeren Membran (2), die ein Pumpvolumen (3) zur Aufnahme und Förderung des medizinischen Fluids (4) ausbildet, wobei die elastomere Membran (2) in einem wenigstens teilweise mit dem medizinischen Fluid (4) befüllten Füllzustand des Pumpvolumens (3) elastisch gedehnt ist und somit einen Förderdruck (p) auf das Pumpvolumen (3) bewirkt, und aufweisend eine einends mit dem Pumpvolumen (3) wirkverbundene Infusionsleitung (8), die andernends zur fluidleitenden Verbindung mit einem Patientenzugang (7) vorgesehen ist und einen Hauptfluidkanal (11) zur Überleitung des medizinischen Fluids (4) von dem Pumpvolumen (3) zu dem Patientenzugang (7) ausbildet, und aufweisend eine mit der Infusionsleitung (8) wirkverbundene mechanische Kontrolleinrichtung (10), die zur Funktionskontrolle der Pumpvorrichtung (1) eingerichtet ist,
**dadurch gekennzeichnet, dass** die Kontrolleinrichtung (10) ein mit dem Hauptfluidkanal (11) wirkverbundenes Differenzdruckmesselement (12) aufweist und derart gestaltet ist, dass ein entlang eines Kanalabschnitts (13) des Hauptfluidkanals (11) ausgebildeter Differenzdruck (Δp) mittels des Differenzdruckmesselements (12) erfassbar und in Abhängigkeit des Differenzdrucks (Δp) eine Förderrate (v) des medizinischen Fluids (4) entlang des Hauptfluidkanals (11) anzeigbar ist,
wobei die Kontrolleinrichtung (10) einen Bypass-Kanal (14) aufweist, in welchem das Differenzdruckelement (12) angeordnet ist und welcher einends mittels eines ersten Kanalabzweigs (15) und andernends mittels eines zweiten Kanalabzweigs (16) jeweils fluiddruckübertragend mit dem Hauptfluidkanal (11) wirkverbunden ist,
wobei das Differenzdruckmesselement (12) wenigstens eine erste Membran (20) und eine zweite Membran (21) aufweist, welche jeweils mittels einer Differenzdruckbeaufschlagung federelastisch auslenkbar sind,
und wobei die Kontrolleinrichtung (10) wenigstens eine fluidgefüllte Fluidkammer (22) aufweist, welche mittels der elastischen Membranen (20, 21) differenzdruckbeaufschlagt ist und in welcher ein Zeigerelement (23) schwimmbeweglich angeordnet ist, welches zur Anzeige der Förderrate (v) in Abhängigkeit des Differenzdrucks (Δp) eingerichtet ist.

2. Infusionsanordnung (A) nach Anspruch 1, **dadurch gekennzeichnet, dass** das Zeigerelement in Form eines Schwimmers (23) oder eines eingefärbten Öltropfens ausgebildet ist.

3. Infusionsanordnung (A) nach Anspruch 1 oder 2, **dadurch gekennzeichnet, dass** die Kontrolleinrichtung (10) ein zwischen dem ersten Kanalabzweig (15) und dem zweiten Kanalabzweig (16) in dem Hauptfluidkanal angeordnetes erstes Fluiddrosselelement (19) aufweist.

4. Infusionsanordnung (A) nach Anspruch 3, **dadurch gekennzeichnet, dass** stromabwärts des zweiten Kanalabzweigs (16) ein zweites Fluiddrosselelement (24) in dem Hauptfluidkanal (11) angeordnet ist.

5. Infusionsanordnung (A) nach Anspruch 3 und 4, **dadurch gekennzeichnet, dass** das erste Fluiddrosselelement (19) im Vergleich zu dem zweiten Fluiddrosselelement (24) eine, vorzugsweise 1,5-fach bis 15-fach, geringere Drosselwirkung aufweist.

## Claims

1. Infusion arrangement (A) for administering a medical fluid (4), having a medical pump apparatus (1) with an elastomeric membrane (2) which forms a pump volume (3) for receiving and delivering the medical fluid (4), wherein the elastomeric membrane (2) is elastically extended in a fill state, filled at least partially with the medical fluid (4), of the pump volume (3) and thereby produces a delivery pressure (p) on the pump volume (3), and having an infusion line (8) which, at one end, is operatively connected to the pump volume (3) and which, at the other end, is provided with a patient access means (7) for fluid-conducting connection and which forms a main fluid channel (11) for transferring the medical fluid (4) from the pump volume (3) to the patient access means (7), and having a mechanical monitoring device (10) which is operatively connected to the infusion line (8) and which is configured for monitoring the functioning of the pump apparatus (1),
**characterized in that** the monitoring device (10) has a differential pressure-measuring element (12) operatively connected to the main fluid channel (11) and is designed such that a differential pressure (Δp) formed along a channel portion (13) of the main fluid channel (11) is able to be detected by means of the differential pressure-measuring element (12) and, in a manner dependent on the differential pressure (Δp), a delivery rate (v) of the medical fluid (4) along the main fluid channel (11) is able to be indicated,
wherein the monitoring device (10) has a bypass channel (14), in which the differential pressure-measuring element (12) is arranged, and which is operatively connected at one end by means of a first channel branch (15), and at the other end by means of a second channel branch (16), to the main fluid channel (11), in each case in fluid pressure-transmitting fashion,
wherein the differential pressure-measuring element (12) has a first membrane (20) and a second membrane (21), which are each able to be deflected in resiliently elastic fashion by means of application of differential pressure,
and wherein the monitoring device (10) has at least one fluid-filled fluid chamber (22), which is pressurized with the differential pressure by means of the elastic membranes (20, 21) and in which an indicator element (23) is arranged so as to be movable in a floating manner, the indicator element (23) being configured for indicating the delivery rate (v) in a manner dependent on the differential pressure (Δp).

2. Infusion arrangement (A) according to Claim 1, **characterized in that** the indicator element is in the form of a float (23) or of a dyed oil drop.

3. Infusion arrangement (A) according to one of Claims 1 or 2, **characterized in that** the monitoring device (10) has a first fluid-throttling element (19) arranged in the main fluid channel between the first channel branch (15) and the second channel branch (16).

4. Infusion arrangement (A) according to Claim 3, **characterized in that** a second fluid-throttling element (24) is arranged in the main fluid channel (11) downstream of the second channel branch (16).

5. Infusion arrangement (A) according to Claims 3 and 4, **characterized in that** the first fluid-throttling element (19) has a throttling action which is lower, preferably 1.5 to 15 times lower, in comparison with the second fluid-throttling element (24).

## Revendications

1. Agencement de perfusion (A) pour l'administration d'un fluide médical (4), présentant un dispositif de pompage médical (1) avec une membrane élastomère (2) qui réalise un volume de pompage (3) pour recevoir et transporter le fluide médical (4), la membrane élastomère (2) étant étirée élastiquement dans un état de remplissage du volume de pompage (3) au moins partiellement rempli du fluide médical (4) et exerçant ainsi une pression de transport (p) sur le volume de pompage (3), et présentant une conduite de perfusion (8) reliée de manière fonctionnelle à une extrémité au volume de pompage (3), qui est prévue à l'autre extrémité pour la liaison de transport de fluide avec un accès de patient (7) et réalise un canal de fluide principal (11) pour le transfert du fluide médical (4) du volume de pompage (3) à l'accès de patient (7), et présentant un appareil de contrôle mécanique (10) relié de manière fonctionnelle à la conduite de perfusion (8), qui est conçu pour le contrôler du fonctionnement du dispositif de pompage (1), **caractérisé en ce que** l'appareil de contrôle (10) présente un élément de mesure de pression différentielle (12) relié de manière fonctionnelle au canal de fluide principal (11) et est conçu de telle sorte qu'une pression différentielle (Δp) réalisée le long d'une section de canal (13) du canal de fluide principal (11) peut être détectée au moyen de l'élément de mesure de pression différentielle (12) et qu'un débit (v) du fluide médical (4) le long du canal de fluide principal (11) peut être affiché en fonction de la pression différentielle (Δp),
l'appareil de contrôle (10) présentant un canal de dérivation (14) dans lequel est agencé l'élément de pression différentielle (12) et qui est relié de manière fonctionnelle au canal de fluide principal (11) par transmission de pression de fluide, à une extrémité au moyen d'une première dérivation de canal (15) et à l'autre extrémité au moyen d'une deuxième dérivation de canal (16),
l'élément de mesure de pression différentielle (12) présentant au moins une première membrane (20) et une deuxième membrane (21) qui peuvent chacune être déviées de manière élastique au moyen d'une sollicitation par pression différentielle,
et l'appareil de contrôle (10) présentant au moins une chambre de fluide remplie de fluide (22) qui est soumise à une pression différentielle au moyen des membranes élastiques (20, 21) et dans laquelle est agencé de manière flottante un élément indicateur (23) qui est conçu pour indiquer le débit (v) en fonction de la pression différentielle (Δp).

2. Agencement de perfusion (A) selon la revendication 1, **caractérisé en ce que** l'élément indicateur est réalisé sous la forme d'un flotteur (23) ou d'une goutte d'huile colorée.

3. Agencement de perfusion (A) selon la revendication 1 ou 2, **caractérisé en ce que** l'appareil de contrôle (10) présente un premier élément d'étranglement de fluide (19) agencé dans le canal de fluide principal entre la première dérivation de canal (15) et la deuxième dérivation de canal (16).

4. Agencement de perfusion (A) selon la revendication 3, **caractérisé en ce qu'**un deuxième élément d'étranglement de fluide (24) est agencé en aval de la deuxième dérivation de canal (16) dans le canal de fluide principal (11).

5. Agencement de perfusion (A) selon les revendications 3 et 4, **caractérisé en ce que** le premier élément d'étranglement de fluide (19) présentant un effet d'étranglement inférieur, de préférence de 1,5 à 15 fois, à celui du deuxième élément d'étranglement de fluide (24).
